# EUROPÄISCHE PATENTSCHRIFT

(11) **EP 4 196 570 B1**
(45) Veröffentlichungstag und Bekanntmachung des Hinweises auf die Patenterteilung: **16.09.2026**
(21) Anmeldenummer: 21765578.6
(22) Anmeldetag: 10.08.2021
(51) Int. Cl.: A61L 27/22, A61L 27/24, A61L 27/36, A61L 27/38, B33Y 80/00, C12N 5/071, C12N 5/077, C12N 5/0775

(54) **AUTOLOGE PRÄVASKULARISIERTE 3D-DRUCKVERFAHREN-ERZEUGTE BRUSTGEWEBE-KONSTRUKTE UND VERFAHREN ZU DEREN HERSTELLUNG**
AUTOLOGOUS, PREVASCULARIZED BREAST TISSUE CONSTRUCTS PRODUCED IN A 3D PRINTING METHOD, AND METHODS FOR PRODUCING SAME
CONSTRUCTIONS DE TISSU MAMMAIRE AUTOLOGUES, PRÉVASCULARISÉES PRODUITES DANS UN PROCÉDÉ D'IMPRESSION 3D, ET LEURS PROCÉDÉS DE PRODUCTION

(30) Priorität: 12.08.2020 DE 102020004900
(43) Veröffentlichungstag der Anmeldung: 21.06.2023
(73) Patentinhaber: Universitätsmedizin der Johannes Gutenberg-Universität Mainz, 55131 Mainz (DE)
(72) Erfinder: HELLER, Martin, 55130 Mainz (DE); BRENNER, Walburgis, Mainz 55124 (DE)
(74) Vertreter: Patentanwälte Dr. Keller, Schwertfeger Partnerschaft mbB
(86) Internationale Anmeldenummer: PCT/EP2021/072240
(87) Internationale Veröffentlichungsnummer: WO 2022/034058

(56) Entgegenhaltungen:
- WO-A1-2015/152954
- WO-A1-2019/151611
- CLEVERSEY CHANTELL ET AL: "3D Printing Breast Tissue Models: A Review of Past Work and Directions for Future Work", MICROMACHINES, vol. 10, no. 8, 27 July 2019 (2019-07-27), pages 501, XP055860302, Retrieved from the Internet <URL:https://www.ncbi.nlm.nih.gov/pmc/articles/PMC6723606/pdf/micromachines-10-00501.pdf> DOI: 10.3390/mi10080501
- BRIAN C. GETTLER ET AL: "Formation of Adipose Stromal Vascular Fraction Cell-Laden Spheroids Using a Three-Dimensional Bioprinter and Superhydrophobic Surfaces", TISSUE ENGINEERING. PART C, METHODS DEC 2008, vol. 23, no. 9, 10 August 2017 (2017-08-10), US, pages 516 - 524, XP055650967, ISSN: 1937-3384, DOI: 10.1089/ten.tec.2017.0056
- SHARATH SIVA SANKARI ET AL: "Human Adipose Tissue Derivatives as a Potent Native Biomaterial for Tissue Regenerative Therapies", TISSUE ENGINEERING AND REGENERATIVE MEDICINE, SPRINGER SINGAPORE, SINGAPORE, vol. 17, no. 2, 17 January 2020 (2020-01-17), pages 123 - 140, XP037080431, ISSN: 1738-2696, [retrieved on 20200117], DOI: 10.1007/S13770-019-00230-X

## Beschreibung

### Technisches Gebiet:

Die vorliegende Erfindung liegt auf dem Gebiet der künstlichen Brustgewebskonstruktion und betrifft ein Verfahren zum dreidimensionalen (3D-)Druck von autologen prävaskularisierten Brustgewebe-Konstrukten sowie ein durch ein solches Verfahren erhältliches Brustgewebe-Konstrukt.

### Stand der Technik:

Brustkrebs ist die häufigste Krebserkrankung der Frau. Die medizinischen Optionen sind vielfältig und richten sich nach Art, Größe, Stadium und histologischem Befund des Krebses. Besonders in frühen Stadien stellt ein adjuvantes Konzept in Form einer operativen Entfernung oder einer Bestrahlung das Verfahren der Wahl für die Behandlung dar. Die chirurgische Entfernung des malignen Befundes in der Brust erfolgt entweder durch eine Mastektomie oder durch die Entfernung von Teilen des Brustgewebes in sano (Rutter, C. E., Park, H. S., Killelea, B. K. & Evans, S. B. Growing Use of Mastectomy for Ductal Carcinoma-In Situ of the Breast Among Young Women in the United States. Ann. Surg. Oncol. 22, 2378-2386 (2015)). Bei der Mastektomie folgt für gewöhnlich die Rekonstruktion des Brustgewebes entweder direkt nach der Tumorresektion oder zu einem späteren Zeitpunkt (Panchal, H. & Matros, E. Current Trends in Postmastectomy Breast Reconstruction. Plast. Reconstr. Surg. 140, 7S-13S (2017); Jeevan, R. et al. Findings of a national comparative audit of mastectomy and breast reconstruction surgery in England. J. Plast. Reconstr. Aesthetic Surg. 67, 1333-1344 (2014)). Entscheidet sich die Patientin für eine Rekonstruktion der Brust, können zwei unterschiedliche Verfahren angewandt werden: Zum einen kann bei einer autologen Rekonstruktion an Körperstellen, wie beispielsweise Bauch, Rücken oder Gesäß, Gewebe entnommen werden, das anschließend das Brustgewebe ersetzt. Dabei werden die umliegende Muskulatur und die Gefäße mit implantiert (z.B. der Latissimus-dorsi-Lappen, myokutane Lappen) oder es kann die Lappenplastik frei durchgeführt werden (Dayan, J. H. & Allen, R. J. Lower Extremity Free Flaps for Breast Reconstruction. Plast. Reconstr. Surg. 140, 77S-86S (2017)). Alternativ kann das Brustgewebe durch heterologe Implantate ersetzt werden. Letztere bestehen entweder vollständig aus Silikon, oder aus einer Silikonhülle, die beispielsweise mit Kochsalzlösung befüllt werden kann, um eine schrittweise Gewebedehnung zu gewährleisten (Yoshida, S. H., Chang, C. C., Teuber, S. S. & Gershwin, M. E. Silicon and Silicone: Theoretical and Clinical Implications of Breast Implants. Regul. Toxicol. Pharmacol. 17, 3-18 (1993)). Die meisten Rekonstruktionen erfolgen durch die Verwendung von Implantaten (Albornoz, C. R. et al. A paradigm shift in U.S. Breast reconstruction: Increasing implant rates. Plast. Reconstr. Surg. 131, 15-23 (2013)), da unter anderem bei nicht allen Patientinnen die Entnahme der benötigten Gewebemasse möglich ist. Des Weiteren bedeutet diese Technik die Entstehung einer zusätzlichen Wunde und der damit einhergehenden Schmerzen und Traumata für die Patientin, was bei einer Rekonstruktion durch Implantate nicht der Fall ist. Außerdem werden Lappenplastiken nicht in allen Krankenhäusern gleich häufig durchgeführt, wodurch der Standort in der Regel entscheidend für die Wahl der Behandlung ist (Alderman, A. K. et al. Patterns and correlates of postmastectomy breast reconstruction by U.S. Plastic surgeons: results from a national survey. Plast. Reconstr. Surg. 127, 1796-803 (2011); Schreuder, K. et al. Hospital organizational factors affect the use of immediate breast reconstruction after mastectomy for breast cancer in the Netherlands. The Breast 34, 96-102 (2017)).

Aufgrund der beschriebenen Herausforderungen wurde in der Vergangenheit nach verschiedenen Ansätzen gesucht, um autologe Gewebe für die Rekonstruktion von verloren gegangenen Brustgeweben künstlich herzustellen. Ein vielversprechender Ansatz stellt in diesem Zusammenhang das Tissue Engineering dar. Mithilfe dieses Verfahrens können z. B. autologe und funktionelle Gewebe aus kleinsten Gewebeproben auf der Basis von bestimmten Trägermatrices wie z.B. Kollagenmembranen in vitro generiert werden. Eine Spezialdisziplin des Tissue Engineering ist das 3D-Bioprinting, mit dem es möglich wird, Zellen direkt in biologisch kompatiblen Biotinten in dreidimensionale Konstrukte zu drucken und so funktionelle Gewebe herzustellen.

Verzweigte Blutarterien und -kapillaren tragen zur Komplexität 3D-gedruckter Organe bei, was für das 3D-Bioprinting eine besondere Herausforderung darstellt. Um ein künstliches Organ oder einen Muskelabschnitt herzustellen, muss die Struktur mit gewebespezifischen Zellen angereichert sein (Zhu W, Qu X, Zhu J, et al. Direct 3D bioprinting of prevascularized tissue constructs with complex microarchitecture. Biomaterials. 2017; 124:106-115; Jia W. Gungor-Ozkerim PS, Zhang YS, et al. Direct 3D bioprinting of perfusable vascular constructs using a blend bioink. Biomaterials. 2016; 106:58-68). Mit Hilfe von Hydrogelen oder anderen biokompatiblen Materialien (Biotinten) und Zellen ist es möglich, präzise 3D-Modelle der Organe herzustellen. Beim 3D-Biodruck unterscheidet man im Wesentlichen zwischen einem gerüstbasierten Druck (scaffold-based printing) und einem gerüstfreien Druck (scaffold-free printing) (Badhshinejad A, D'Souza RM. A brief comparison between available bio-printing methods. In 2015 IEEE Great Lakes biomedical conference (GLBC) 1-3 (IEEE, 2015).2015). Es wurden verschiedene Ansätze für den gerüstbasierten Biodruck entwickelt, beispielsweise indem zuerst ein 3D-Gerüst aus Biomaterialien hergestellt wird und anschließend ein Druck der Zellen in die Struktur erfolgt. Daneben sind auch Verfahren verfügbar, bei denen die Gerüststruktur und die Zellen gleichzeitig gedruckt werden. Bei einem gerüstfreien Druck beinhaltet die Biotinte unterschiedliche Zellen oder Gewebe-Spheroide, um diese zusammen mit der Biotinte direkt zu drucken (Ong CS, Fukunishi T. Zhang H, et al. Biomaterial-free three-dimensional bioprinting of cardiac tissue using human induced pluripotent stem cell derived cardiomyocytes. Sci Rep. 2017;7(1):4566).

Zum Druck von vaskulären Strukturen werden im Wesentlichen zwei Ansätze beim 3D-Druck verfolgt, zum einen der Direktdruck der Gefäße, zum anderen der indirekte Druck von Gerüstmaterialien (z.B. Gelatine, Kollagen), die in einem Hydrogel vermengt werden (Datta P, Ayan B.Ozbolat IT. Bioprinting for vascular and vascularized tissue biofabrication. Acta Biomater. 2017; 51:1-20). Wenn sich die Hydrogelgerüst-Struktur verfestigt, verbleibt eine hohle Gefäßstruktur, auf der später mit Endothelzellen kultiviert wird. Dieses Verfahren wird auch Microextrusion genannt und hat den Vorteil, dass stabile Gefäßstrukturen und viele unterschiedliche Gerüststrukturen miteinander kombinierbar sind. Zum Druck von Gefäßstrukturen werden häufig native Materialien wie Fibrin oder Kollagen eingesetzt (Hinton TJ. Jallerat Q. Palchesko RN, et al. Three-dimensional printing of complex biological structures by freeform reversible embedding of suspended hydrogels. Sci Adv. 2015;1(9): el500758). Kollagen I kann beispielsweise mit adipöser Stromal Vascular Fraction (SVF) als gelbildende Biomatrix gedruckt werden, woraus zellenthaltene Sphäroide erhalten werden (Brian C. Gettler et al., Formation of Adipose Stromal Vascular Fraction Cell-Laden Spheroids Using a Three-Dimensional Bioprinter and Superhydrophobic Surface; Tissue Engineering. Part C, 2017, 23, 516-524). Daneben gibt es auch PEG-Fibrinogen-basierte Gerüststrukturen zur Entwicklung von dreidimensionalem Herzgewebe-Konstrukten, die aus von pluripotenten Zellen abgeleiteten Kardiomyozyten bestehen (Maiullari F. Costantini M, Milan M. et al. A multi-cellular 3D bio-printing approach for vascularized heart tissue engineering based on HUVECs and iPSC-derived cardiomyocytes. Sci Rep. 2018:8(1):1-15). Bei Brustkrebs sind bereits verschiedene Ansätze und einige Methoden zum 3D-Druck auf Basis von Brustgewebemodellen bekannt (Cleversey Chantell et al., 3D Printing Breast Tissue Models: A Review of Past Work and Directions for Future Work; Micromachines 2019, 10, 501).

Es gibt unterschiedlichste Fettgewebederivate wie autologes Fetttransplantat, SVF, Stammzellen und Präadipozyten für regenerierende Therapien, wobei SVF Muskelzellen, Fibroblasten, Endothelzellen und aus Fettgewebe stammende Stammzellen umfassen (Sharath Siva Sankari et al., Human Adipose Tissue Derivatives as a Potent Native Biomaterial for Tissue Regenerative Therapies; Tissue Engineering and Regenerative Medicine, 2020, 17, 123-140).

Die WO 2015/152954 A1 beschreibt ein Verfahren zur Herstellung von künstlichem Gewebe, u.a. eines künstlichen 3D-Brustgewebes, um Modelle für die Krebstherapie bereitzustellen. Das Verfahren verwendet hierzu eine Extrusionsstoff-haltige Biotinte, die Bindegewebszellen und eine weitere Extrusionsstoff-haltige Biotinte, die Krebszellen umfasst. Nach einer Inkubationsperiode in einer Zellkultur wird der Extrusionsstoff entfernt und den Zellen ermöglicht, ein dreidimensionales biologisches Tumor-Modell zu bilden.

Für den Druck von prävaskularisierten Strukturen können unterschiedliche Biotinten zum Einsatz kommen, beispielsweise zellfreie Biotinte oder zellhaltige Biotinte, die für den Inkjet-Biodruck, Extrusions-Biodruck oder gepulsten Laser-Druck einsetzbar sind. Bei Extrusions-Biodruckern hat sich Alginat-enthaltende Biotinte als vorteilhaft herausgestellt (van Duinen V, Trietsch S.J. Joore J, Vulto P, Hankemeier T. Microfluidic 3D cell culture: from tools to tissue models. Curr Opin Biotechnol. 2015;35: 118-126). Zur Dispersion von Fibroblasten kommt häufig Zell-verklebende GelMA-Biotinte zur Ausbildung von Kanalstrukturen zum Einsatz. Die reinen zellhaltigen Biotinten wiederum ermöglichen eine Drucklösung mit geringer Viskosität, so dass sie in einer Vielzahl von Biodrucksystemen einsetzbar sind. Allerdings benötigen solche Biotinten ausreichende biologische Signale (z.B. zelladhäsive Matrix, Trägerzelltypen), um die Zell-Zell-Interaktionen nach dem Druck zu stabilisieren.

Die WO 2019/122351 A1 beschreibt Biotinten auf der Basis von Nanocellulose bzw. einem Polysaccharid-Hydrogel und einem humanen gewebespezifischen extrazellulären Matrix (ECM)-Material, wobei der 3D-Druck unter physiologischen Bedingungen erfolgt.

Das Herstellen einer dreidimensionalen Rekonstruktion der Brust, insbesondere nach einer Mammakarzinom-Resektion, stellt daher eine besondere Herausforderung dar. Gleichwohl überwiegen die Vorteile gegenüber den klassischen Methoden, insbesondere der plastischen Rekonstruktion, welche üblicherweise nach der operativen Resektion von Tumorgewebe aus der Brust bzw. einer vollständigen Brustentfernung angewendet wird. Dabei spielen neben der körperlichen und psychischen Belastung für die Patientinnen vor allem auch ästhetisch ansprechende Ergebnisse eine entscheidende Rolle.

### Darstellung der Erfindung:

Vor diesem Hintergrund ist es daher Aufgabe der vorliegenden Erfindung, ein verbessertes 3D-Druckverfahren zur Herstellung von Brustgewebe-Konstrukten bereitzustellen, bei dem eine Rekonstruktion der Brust nach einer Tumor-Resektion möglich ist, ohne dass sekundäre Traumata erzeugt werden. Diese Aufgabe wird gelöst durch ein Verfahren mit den Merkmalen des Anspruchs 1. Bevorzugte Ausführungsvarianten finden sich in den Unteransprüchen wieder.

Das erfindungsgemäße Verfahren basiert auf komplexen autologen Brustgewebe-Konstrukten, die über einen 3D-Biodruck erzeugt werden. Verfahrensgemäß werden autologe, d.h. von der Patientin entnommene, Zellen verwendet, wodurch Abstoßungsreaktionen auf ein Minimum reduziert werden können. Die erfindungsgemäß eingesetzten Zellen ermöglichen ein organtypisches Mikromilieu, was letztendlich für eine zügige Gefäßbildung in dem künstlichen Brustgewebe-Konstrukt sorgt. Die Herstellung der Brust(fett)gewebe-Konstrukte erfolgt anhand einer komplexen Trikultur aus primären mesenchymalen Stammzellen bzw. (Prä-)Adipozyten, Fibroblasten und endothelialen Progenitorzellen, die aus kleinsten Gewebeproben oder Blut der Patientin isoliert werden. Sekundäre Traumata und damit assoziierte Komorbiditäten, die bei der Entnahme autologer Gewebetransplantate auftreten, werden auf ein Minimum reduziert.

Eine Kultur vorbehandelter adipöser mesenchymaler Stammzellen, Fibroblasten und endothelialer Progenitorzellen wird mit einer aus Biopolymeren bestehenden Biotinte vermengt und zu komplexen prävaskularisierten Bruststrukturen gedruckt. Dabei erfolgt entweder der Druck von gefäßähnlichen dreidimensionalen Strukturen, oder die Kapillarbildung wird nach dem Druck durch Kultivierung induziert. Erfindungsgemäß werden die Zellen der Trikultur vor dem Druck mit Wachstumsmedium vorbehandelt, damit die endothelialen Progenitorzellen zu Endothelzellen und die adipösen mesenchymalen Stammzellen zu Adipozyten differenzieren. Die Gefäßstrukturen des Brustgewebekonstruktes werden vorzugsweise mit gefäßbildenden Zellen, bevorzugt Endothelzellen, gedruckt.

Das erfindungsgemäße Verfahren zum 3D-Druck von autologen prävaskularisierten Brustgewebe-Konstrukten umfasst somit die folgenden Schritte:
(i) Bereitstellen einer Trikultur, bestehend aus adipösen mesenchymalen Stammzellen, Fibroblasten und endothelialen Progenitorzellen, die aus autologen Zellen einer Patientin stammen,
(ii) Vermengen der Trikultur-Zellen mit einer aus Biopolymeren bestehenden Biotinte, wobei die Biotinte Kollagen I umfasst,
(iii) Drucken von dreidimensionalen prävaskulärisierten Strukturen des Brustgewebe-Konstrukts unter Verwendung der mit der Trikultur versetzten Biotinte aus Schritt (ii), wobei die Zellen der Trikultur vor dem Druck mit Wachstumsmedien vorbehandelt werden, so dass die endothelialen Progenitorzellen zu Endothelzellen und die adipösen mesenchymalen Stammzellen zu Adipozyten differenzieren und wobei nach dem 3D-Druck die Entwicklung gefäßähnlicher Strukturen durch das in der Biotinte enthaltene Kollagen I induziert wird.

Vorzugsweise besteht die Biotinte aus Biopolymeren, wie Cellulose, Alginat, Mannitol, Gelatine-Methacrylat und/oder Kollagen I. Die Erfinder konnten zeigen, dass die Kultivierung und das Drucken der verschiedenen Zelltypen der Trikultur in Kollagen-basierten Biotinten möglich sind, was die Voraussetzung für die Bildung von kapillarähnlichen dreidimensionalen Strukturen des Brustgewebes ist. Eine zusätzliche Option ist die Verwendung selbst extrahierter extrazellulärer Matrix aus Fettgewebe (adECM; adipose derived extracellular matrix), das - wie auch die Zellen - autolog gewonnen und beispielsweise in einer Zusammensetzung oder in Mischung mit anderen genannten Tinten verwendet werden kann. Die in der Erfindung verwendeten Zellen zeigten eine hohe Viabilität unter Verwendung der adECM. Somit wurde zum ersten Mal gezeigt, dass die Konstruktion von Brust(fett)gewebe über eine mit Wachstumsmedium vorbehandelte Zellkultur, bestehend aus adipösen mesenchymalen Stammzellen, Fibroblasten und endothelialen Progenitorzellen, möglich ist, um autologe Brustgewebe-Konstrukte zu drucken. Das Verfahren ermöglicht die Herstellung von prävaskularisierten Brustfettgewebe-Konstrukten, d.h. Gewebekonstrukten, welche bereits kapillarähnliche Strukturen, die aus Endothelzellen bestehen, besitzen. Die Prävaskularisierung kann beispielsweise anhand von zwei Methoden in das Gewebeäquivalent integriert werden. Zum einen werden primäre Endothelzellen zusammen mit Fibroblasten und mesenchymalen Stammzellen in der aus Biopolymeren bestehenden Biotinte direkt in das Konstrukt mittels 3D-Bioprinting gedruckt, was zur Bildung eines fein verzweigten Netzwerks aus kapillarähnlichen Strukturen führt. Zum anderen werden größere Gefäße in Form eines Kanal- oder Röhrensystems in das Gewebekonstrukt gedruckt. Dies erfolgt mit den gefäßbildenden Endothelzellen. Ausgehend von diesen gedruckten Gefäßkanälen kann in der Folge ein feinverzweigtes Gefäßnetz entstehen, welches schließlich das gesamte Gewebekonstrukt durchspannt. Durch die Kombination der beiden Methoden entstehen Gewebekonstrukte, die mit großen und kleinen Gefäßnetzwerken durchzogen sind. Die Prävaskularisierung ist essentiell für eine ausreichende und rechtzeitige Anbindung an das Gefäßsystem der Empfängerin nach einer Transplantation, um ein erfolgreiches Einhalten bzw. Einwachsen sicherzustellen und alle Bereiche der Gewebekonstrukte oder die dort enthaltenen Zellen ausreichend mit Sauerstoff und Nährstoffen zu versorgen.

Die einzelnen Komponenten der Biotinte können in ihrer Zusammensetzung und Konzentration variieren. Vorzugsweise enthält die erfindungsgemäße Biotinte Kollagen I, damit die Bildung von Gefäßstrukturen induziert und die Vitalität der Zellen verbessert werden kann. Vorzugsweise erfolgt eine mehrtägige, bevorzugt 7-tägige Co-Kultur in einer Kollagen I-haltigen Biotinte zur Bildung von gefäßähnlichen Strukturen. Die Viabilität einer Co-Kultur von Endothelzellen und Fibroblasten in einer Kollagen I-haltigen im Vergleich zu einer Kollagen I-freien Biotinte kann beispielsweise über einen **MTT-** oder Alamarblue-Assay erfolgen. Diese Testsysteme dienen der Bestimmung der metabolischen Aktivität von Zellen, die unter bestimmten Bedingungen mit der Zellviabilität korreliert. Der Nachweis der Zellviabilität beruht auf einer Reduktion des gelblichen, wasserlöslichen Farbstoffes 3-(4,5-Dimethyltiazol-2-yl)-2,5-diphenyltetrazoliumbromid (MTT) in ein blau-violettes, wasserunlösliches Formazan bzw. im Falle des Alamarblue-Tests auf einer Reduktion des blauen Resazurin zu pink-farbenem Resofurin.

Der Einsatz von mesenchymalen Stammzellen bzw. Vorläuferzellen in einem komplexen Brustfettgewebe-Äquivalent stellt einen zentralen Baustein der vorliegenden Erfindung dar, da es sich um autologe Zellen handelt, die aus kleinsten Gewebeproben bzw. Blut von Patienten isoliert werden und für das erfindungsgemäße Verfahren zur Verfügung gestellt werden. Die Entnahme ist hierbei so unbedeutend, dass sekundäre Traumata oder damit assoziierte Komorbiditäten, die bei der Entnahme autologer Gewebetransplantate auftreten, gänzlich vermieden werden. Mesenchymale Stammzellen sind in adulten Geweben einschließlich des Knochenmarks und des Fettgewebes vorhanden. Die Stammzellen sind anhand ihrer Oberflächenmarker CD105, CD73 und CD90 bei fehlender Expression von CD34, CD45, CD14 oder CD11b, CD19 und CD79α oder HLA DR nachweisbar. Sie haben die Fähigkeit, sich zu Adipozyten, aber auch zu Osteoblasten oder Chondroblasten zu differenzieren.

Die in der Trikultur eingesetzten autologen endothelialen Progenitorzellen stammen vorzugsweise aus dem Blut der Patientin. Die autologen Fibroblasten werden vorzugsweise aus einer kleinen Mundschleimhaut-Biopsie der Patientin gewonnen. Die autologen mesenchymalen Stammzellen stammen vorzugsweise aus dem Fettgewebe der Patientin. Die so gewonnenen Zellen der Trikultur werden vor dem Druck zunächst isoliert, separat expandiert und zur Differenzierung über entsprechende Wachstumsfaktoren enthaltende Medien angeregt.

In einer bevorzugten Ausführungsform werden bereits differenzierte Endothelzellen zur Prävaskularisierung von Gewebekonstrukten verwendet. In einem unabhängigen Vorversuch wurden im Rahmen der Erfindung auch mikrovaskuläre Endothelzellen, welche aus der Vorhaut junger männlicher Patienten isoliert wurden, verwendet. Allerdings ist dadurch das Einsatzgebiet der Brustfettgewebe-Konstrukte stark begrenzt und für Patientinnen nach Brustoperationen nicht geeignet, so dass die erfindungsgemäß eingesetzten endotheliale Progenitorzellen für den 3D-Druck des prävaskularisierten Gewebekonstrukts besonders vorteilhaft sind. Ähnlich den differenzierten Endothelzellen, sind endotheliale Progenitorzellen in der Lage, komplexe Gefäßstrukturen auszubilden. Für die Prävaskularisierung von Brust(fett)gewebe-Konstrukten wird eine heterogene, zirkulierende Zellpopulation endothelialer Progenitorzellen eingesetzt, die bevorzugt aus "späten" endothelialen Progenitorzellen (späte EPCs) bestehen. Die weitaus größere Sub-Population früher EPCs wiederum fördert wahrscheinlich, vermittelt durch parakrine Effekte, endotheliale Reparaturprozesse. Für eine Prävaskularisierung von Brustgewebe spielen diese Zellen eher eine geringere Rolle.

Die endothelialen Progenitorzellen, die aus dem Blut einer Patientin stammen, werden zunächst kultiviert und anschließend auf eine Gelatine-beschichtete Kulturoberfläche und ein spezielles Selektionsmedium umgestellt. Bei dem Selektionsmedium handelt es sich vorzugsweise um ein endotheliales Zell-Wachstumsmedium, wie beispielsweise das EGM^{™} Bullet-Kit der Firma Lonza. Die adipösen mesenchymalen Stammzellen werden beispielsweise mittels AdipoMAX (Sigma-Aldrich) zur Differenzierung zu Adipozyten angeregt und durch Anti-CD34-gekoppelte Magnetbeads selektioniert. Die Isolierung von Fibroblasten erfolgt anhand bekannter Methodik, gezeigt anhand künstlicher prävaskularisierter Schleimhaut-Äquivalente (Heller et al., Tissue engineered pre-vascularized buccal mucosa equivalents utilizing a primary triculture of epithelial cells, endothelial cells and fibroblasts. Biometarials 77:2017-15 (2016)).

Die Differenzierung und Vitalität der Endothelzellen ist ein wesentlicher Faktor für eine erfolgsversprechende Durchführung des erfindungsgemäßen Verfahrens und das daraus hervorgehende Produkt. Es ist daher in einer bevorzugten Ausführungsvariante vorgesehen, dass die Endothelzellen nicht direkt als Suspension gedruckt werden, sondern vorab als Spheroide oder auf Microcarriern (z.B. auf Gelatine-beschichtete Microcarrier). Die Kultivierung auf Microcarriern erhöht die Vitalität und Differenzierung der Zellen. Dadurch erlangen die Zellen bereits vor dem 3D-Biodruck eine dreidimensionale Kulturstruktur, welche die Gefäßbildung nach dem Druck unterstützt.

Über das erfindungsgemäße 3D-Druckverfahren kann ein autologes prävaskularisiertes Brustgewebe-Konstrukt hergestellt werden, das eine dreidimensionale Struktur aus mehreren unterschiedlichen Zelltypen umfasst, bestehend aus Endothelzellen, die aus endothelialen Progenitorzellen differenziert wurden, Adipozyten, die aus adipösen mesenchymalen Stammzellen differenziert wurden, und Fibroblasten.

Die Erfindung bietet die Möglichkeit, autologes Brust(fett)gewebe herzustellen, ohne die ansonsten üblichen sekundären Operationstraumata zu erzeugen. Die Entnahme kleinster Gewebeproben oder Blut für die Isolation der beim erfindungsgemäßen Verfahren eingesetzten autologen Zellen der Trikultur reduziert die zusätzliche Belastung für die Patientinnen auf ein Minimum. Des Weiteren können Gewebekonstrukte in beliebiger Größe hergestellt werden, wodurch auch große Gewebedefekte, wie sie z.B. bei einer vollständigen Brustentfernung entstehen, rekonstruiert werden können. Nach dem Herstellungsprozess der Gewebekonstrukte sind diese steril und können direkt verwendet werden. Dabei können durch die verschiedenen wählbaren Hardware-Parameter eines 3D-Biodrucks unterschiedliche Größen und Formen hergestellt werden.

### Kurze Beschreibung der Zeichnungen:

Weitere Merkmale und Vorteile der vorliegenden Erfindung werden deutlich anhand der nachfolgenden Beschreibung bevorzugter Ausführungsbeispiele unter Bezugnahme auf die beiliegenden Abbildungen. Es zeigen:
- Fig. 1: die Bildung von gefäßähnlichen Strukturen nach einer 7-tägigen Kultur in einer Kollagen I-haltigen Biotinte,
- Fig. 2: die Viabilität einer Endothelzell-Fibroblasten-Co-Kultur in einer Kollagen I-haltigen Biotinte im Vergleich zu einer Kollagen I-freien Biotinte,
- Fig. 3: die Kultivierung von Endothelzellen auf Microcarriern zur Erhöhung der Vitalität und Differenzierung der Zellen.

### Wege zur Ausführung der Erfindung:

Die nachfolgenden Ausführungsbeispiele dienen der Veranschaulichung der Erfindung.

Keinesfalls ist die Erfindung auf diese Ausführungsbeispiele beschränkt. Die Erfindung umfasst auch Kombinationen einzelner Ausführungsformen oder beliebige Kombinationen von Merkmalen einzelner Ausführungsvarianten.

Bei den nachfolgenden Ausführungsbeispielen werden zwei verschiedene Ansätze für die Herstellung der prävaskularisierten Gewebekonstrukte verfolgt, welche entweder auf einem 1-Kanal-, oder 2-Kanal-Druck basieren.

Für die Herstellung eines fein verzweigten Netzwerkes aus kapillarähnlichen Strukturen innerhalb kleinerer Gewebekonstrukte werden für den ersten Ansatz (1-Kanal) die abgelösten Zellen zu gleichen Teilen zusammen in Zellmedium überführt, sodass eine Trikultur mit einer Gesamt-Zellkonzentration von 10⁶ Zellen/ml erreicht wird. Anschließend wird die Zellsuspension mit der Kollagen-basierten Biotinte im Verhältnis 1:10 mittels Luer-Lock Spritze und Luer-Lock Adapter gemischt und in eine Druck-Kartusche überführt. Nach Einsetzen der Kartusche werden die 3D-Konstrukte mit einem Druck von 9-15 kPa und einer 25G Spitze in eine sterile Well-Platte in einem Kanal gedruckt.

Für den zweiten Ansatz werden größere Gefäße in Form eines Kanal- oder Röhrensystems in das Gewebekonstrukt (1-Kanal und/oder 2-Kanal) gedruckt. Hierfür werden die Zellen nach Ablösen aus den Kulturgefäßen zunächst getrennt. Dabei werden mesenchymale Stammzellen bzw. Adipozyten und Fibroblasten (Bikultur) zusammen in eine Zellsuspension und Endothelzellen (Monokultur) in eine weitere Suspension mit jeweils einer Gesamt-Zellkonzentration von 10⁶ Zellen/ml überführt. Anschließend werden die verschiedenen Zell-Suspensionen (Mono- und Bikultur) mit der Kollagen-basierten Biotinte im Verhältnis 1:10 wie oben beschrieben gemischt und in zwei Druck-Kartuschen aufgeteilt. Für das hier beschriebene Vorgehen werden ebenfalls 25G Druckspitzen verwendet. Für den 1-Kanal Druck wird zunächst ein Grundgerüst aus der Bikultur gedruckt um eine Bindegewebestruktur herzustellen, welche röhrenartige Aussparungen bzw. poröse Strukturen aufweist. Im Anschluss wird dann das Endothelzell-Biotinten-Gemisch für das Drucken der Gefäßstrukturen verwendet. Im 2-Kanal-System wird das Grundgerüst aus der Bikultur und simultan hierzu unter Verwendung des zweiten Kanals Gefäßstrukturen mit der Endothelzell-Monokultur gedruckt. Die Ergebnisse sind in den nachfolgenden Abbildungen zusammengefasst.

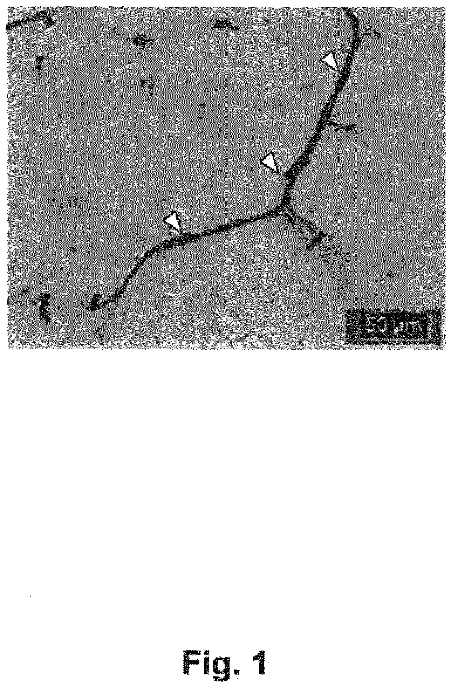

Fig. 1 stellt die Morphologie von Endothelzellen auf einer Kollagen I-basierten Biotinte dar, visualisiert durch eine CD31-Färbung (hier schwarz). Zu sehen sind gefäßähnliche Strukturen (weiße Pfeile), die sich nach 7-tägiger Co-kultur von Endothelzellen mit Fibroblasten in einer Kollagen I-haltigen Biotinte ausbilden.

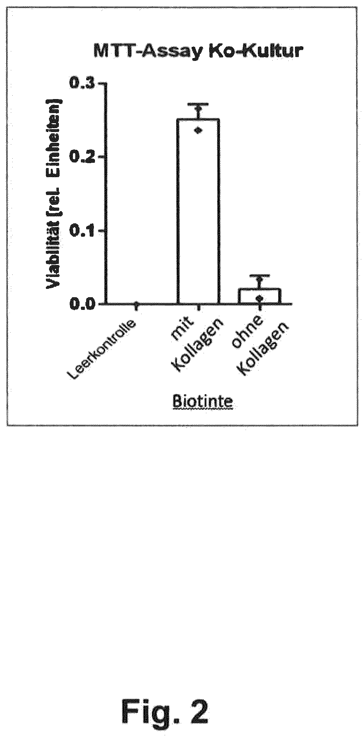

Fig. 2 zeigt die Viabilität einer Endothelzell/Fibroblasten-Co-kultur in einer Kollagen I-haltigen im Vergleich zu einer Kollagen I-freien Biotinte, nachgewiesen in einem MTT-Assay. Der Kollagen I-Anteil in der Biotinte ist ausschlaggebend für eine hohe Viabilität.

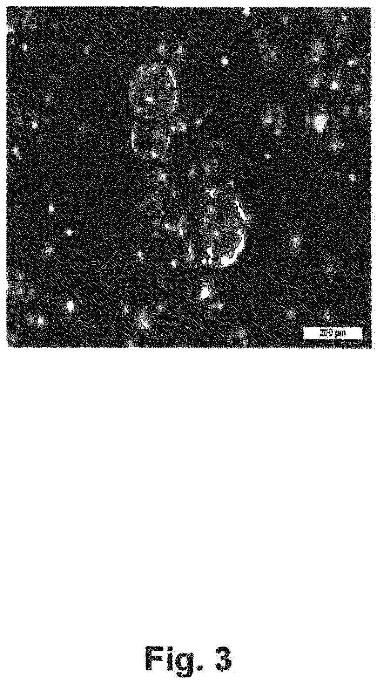

Fig. 3 zeigt Endothelzellen kultiviert auf Gelatine-beschichteten Microcarriern, zu erkennen als helle Färbung (CD31). Durch die Adhärenz auf Microcarriern behalten die Endothelzellen eine hohe Vitalität und Differenzierung der Zellen.

## Patentansprüche

1. Verfahren zum 3D-Druck von autologen prävaskularisierten Brustgewebe-Konstrukten, umfassend die Schritte:
(i) Bereitstellen einer Trikultur, bestehend aus adipösen mesenchymalen Stammzellen, Fibroblasten und endothelialen Progenitorzellen, die aus autologen Zellen einer Patientin stammen,
(ii) Vermengen der Trikultur-Zellen mit einer aus Biopolymeren bestehenden Biotinte, wobei die Biotinte Kollagen I umfasst,
(iii) Drucken von dreidimensionalen prävaskularisierten Strukturen des Brustgewebe-Konstrukts unter Verwendung der mit der Trikultur versetzten Biotinte aus Schritt (ii), wobei die Zellen der Trikultur vor dem Druck mit Wachstumsmedien vorbehandelt werden, so dass die endothelialen Progenitorzellen zu Endothelzellen und die adipösen mesenchymalen Stammzellen zu Adipozyten differenzieren und wobei nach dem 3D-Druck die Entwicklung gefäßähnlicher Strukturen durch das in der Biotinte enthaltene Kollagen I induziert wird.

2. Verfahren nach Anspruch 1, **dadurch gekennzeichnet, dass** die Entwicklung gefäßähnlicher Strukturen nach dem 3D-Druck mit Kollagen I induziert wird.

3. Verfahren nach Anspruch 1, **dadurch gekennzeichnet, dass** der 3D-Druck in einem 1-Kanal-System und/oder einem 2- Kanal-System erfolgt.

4. Verfahren nach einem der vorhergehenden Ansprüche, **dadurch gekennzeichnet, dass** die Gefäßstrukturen des Brustgewebe-Konstruktes mit gefäßbildenden Zellen, vorzugsweise Endothelzellen gedruckt werden.

5. Verfahren nach Anspruch 4, **dadurch gekennzeichnet, dass** es sich bei den Endothelzellen um differenzierte Endothelzellen oder mikrovaskuläre Endothelzellen handelt.

6. Verfahren nach einem der vorhergehenden Ansprüche, **dadurch gekennzeichnet, dass** die Biotinte Cellulose, Alginat, Mannitol, Gelatine-Methacrylat und/oder Kollagen I enthält.

7. Verfahren nach Anspruch 1, **dadurch gekennzeichnet, dass** es sich bei den endothelialen Progenitorzellen um späte endotheliale Progenitorzellen aus dem Blut der Patientin handelt.

8. Verfahren nach Anspruch 1, **dadurch gekennzeichnet, dass** die endothelialen Progenitorzellen erhältlich sind durch eine mehrtägige Kultivierung der von dem Blut entnommenen Zellen und eine Umstellung auf eine Gelatine-beschichteten Kulturoberfläche mit einem Selektionsmedium.

9. Verfahren nach einem der vorhergehenden Ansprüche, **dadurch gekennzeichnet, dass** die Biotinte in einer Zusammensetzung enthalten ist, die selbst extrahierte extrazelluläre Matrix aus Fettgewebe (adECM) enthält.

10. Verfahren nach Anspruch 1, **dadurch gekennzeichnet, dass** die adipösen mesenchymalen Stammzellen aus einer vorliegenden Fettgewebeprobe der Patientin durch anti-CD34-gekoppelte Magnetbeads selektioniert werden.

11. Verfahren nach Anspruch 1, **dadurch gekennzeichnet, dass** die Fibroblasten aus einer vorliegenden Mundschleimhaut-Probe der Patientin selektioniert werden.

12. Verfahren nach Anspruch 1, **dadurch gekennzeichnet, dass** die Endothelzellen vor dem Druck als Spheroide oder auf Microcarriern kultiviert werden.

## Claims

1. A method for 3D printing autologous pre-vascularized breast tissue constructs, comprising the steps of:
(i) providing a triculture consisting of adipose mesenchymal stem cells, fibroblasts, and endothelial progenitor cells derived from a patient's autologous cells,
(ii) mixing the triculture cells with an organic ink consisting of biopolymers, wherein the organic ink comprises collagen I,
(iii) printing three-dimensional, pre-vascularized structures of the breast tissue construct using the organic ink mixed with the triculture from step (ii),
wherein the cells of the triculture are pretreated with growth media prior to printing so that the endothelial progenitor cells differentiate to endothelial cells and the adipose mesenchymal stem cells differentiate to adipocytes, and wherein, after 3D printing, the development of vascular-like structures is induced by the collagen I contained in the organic ink.

2. The method according to claim 1, **characterized in that** the development of vascular-like structures is induced with collagen I after 3D printing.

3. The method according to claim 1, **characterized in that** the 3D printing is performed in a 1-channel system and/or a 2-channel system.

4. The method according to one of the preceding claims, **characterized in that** the vascular structures of the breast tissue construct are printed with vasculogenic cells, preferably endothelial cells.

5. The method according to claim 4, **characterized in that** the endothelial cells are differentiated endothelial cells or microvascular endothelial cells.

6. The method according to one of the preceding claims, **characterized in that** the organic ink contains cellulose, alginate, mannitol, gelatin methacrylate, and/or collagen I.

7. The method according to claim 1, **characterized in that** the endothelial progenitor cells are late-stage endothelial progenitor cells derived from the patient's blood.

8. The method according to claim 1, **characterized in that** the endothelial progenitor cells are obtainable by cultivating the cells obtained from the blood for several days and by switching to a gelatin-coated culture surface with a selection medium.

9. The method according to one of the preceding claims, **characterized in that** the organic ink is contained in a composition that itself contains extracted extracellular matrix from adipose tissue (adECM).

10. The method according to claim 1, **characterized in that** the adipose mesenchymal stem cells are selected from an existing adipose tissue sample from the patient using anti-CD34-conjugated magnetic beads.

11. The method according to claim 1, **characterized in that** the fibroblasts are selected from an existing oral mucosa sample from the patient.

12. The method according to claim 1, **characterized in that** the endothelial cells are cultivated as spheroids or on microcarriers prior to printing.

## Revendications

1. Procédé pour l'impression 3D de constructions de tissu mammaire autologues prévascularisées, comprenant les étapes :
(i) Fournir une triculture, composée de cellules souches mésenchymateuses adipeuses, de fibroblastes et de cellules progénitrices endothéliales, qui proviennent de cellules autologues d'une patiente,
(ii) Mélanger les cellules de triculture avec une bioencre composée de biopolymères, la bioencre comprenant du collagène I,
(iii) Imprimer des structures tridimensionnelles prévascularisées de la construction de tissu mammaire en utilisant la bioencre additionnée de triculture de l'étape (ii), les cellules de triculture étant prétraitées avec des milieux de croissance avant l'impression, de sorte que les cellules progénitrices endothéliales différencient en cellules endothéliales et les cellules souches mésenchymateuses adipeuses différencient en adipocytes, et où, après l'impression 3D, le développement de structures de type vasculaire est induit par le collagène I contenu dans la bioencre.

2. Procédé selon la revendication 1, **caractérisé en ce que** le développement de structures de type vasculaire après l'impression 3D est induit avec du collagène I.

3. Procédé selon la revendication 1, **caractérisé en ce que** l'impression 3D est réalisée dans un système à canal unique et/ou un système à deux canaux.

4. Procédé selon l'une des revendications précédentes, **caractérisé en ce que** les structures vasculaires de la construction de tissu mammaire sont imprimées avec des cellules angio'-génératrices, de préférence des cellules endothéliales.

5. Procédé selon la revendication 4, **caractérisé en ce que** les cellules endothéliales sont des cellules endothéliales différenciées ou des cellules endothéliales microvasculaires.

6. Procédé selon l'une des revendications précédentes, **caractérisé en ce que** la bioencre contient de la cellulose, de l'alginate, du mannitol, du méthacrylate de gélatine et/ou du collagène I.

7. Procédé selon la revendication 1, **caractérisé en ce que** les cellules progénitrices endothéliales sont des cellules progénitrices endothéliales tardives provenant du sang de la patiente.

8. Procédé selon la revendication 1, **caractérisé en ce que** les cellules progénitrices endothéliales sont obtenues par une culture de plusieurs jours des cellules prélevées du sang et un passage sur une surface de culture recouverte de gélatine avec un milieu de sélection.

9. Procédé selon l'une des revendications précédentes, **caractérisé en ce que** la bioencre est comprise dans une composition qui contient une matrice extracellulaire auto-extraite de tissu adipeux (adECM).

10. Procédé selon la revendication 1, **caractérisé en ce que** les cellules souches mésenchymateuses adipeuses sont sélectionnées à partir d'un échantillon de tissu adipeux de la patiente à l'aide de billes magnétiques couplées à l'anti-CD34.

11. Procédé selon la revendication 1, **caractérisé en ce que** les fibroblastes sont sélectionnés à partir d'un échantillon de muqueuse buccale de la patiente.

12. Procédé selon la revendication 1, **caractérisé en ce que** les cellules endothéliales sont cultivées avant l'impression sous forme de sphéroïdes ou sur des microporteurs.
